Europäisches Patentamt

(19) European Patent Office

Office européen des brevets

(11) Publication number: **0 319 542 B1**

# (12) EUROPEAN PATENT SPECIFICATION

(45) Date of publication of patent specification: **08.12.93**  (51) Int. Cl.⁵: **G01N  33/569**, C12Q 1/70

(21) Application number: **87905501.0**

(22) Date of filing: **29.07.87**

(86) International application number:
**PCT/US87/01816**

(87) International publication number:
**WO 88/01387 (25.02.88 88/05)**

---

(54) **TESTING FOR THE HUMAN B LYMPHOTROPIC VIRUS (HBLV).**

---

(30) Priority: **11.08.86 US 895463**

(43) Date of publication of application:
**14.06.89 Bulletin  89/24**

(45) Publication of the grant of the patent:
**08.12.93 Bulletin  93/49**

(84) Designated Contracting States:
**AT BE CH DE FR GB IT LI LU NL SE**

(56) References cited:
**WO-A-84/04327**
**WO-A-86/02930**

**SCIENCE, vol. 234, 31 October 1986; S.Z. SALAHUDDINet al., pp. 596-601&NUM;**

**MICROBIOLOGY, 3rd ed., chapter 55, 1980, Harper & Row (Publ.), Hagerstown; pp. 1061-1076&NUM;**

(73) Proprietor: **THE UNITED STATES OF AMERICA as represented by the Secretary United States Department of Commerce**

National Technical Information Service, Office of Government Inventions and Patents,
5285 Port Royal Road
Springfield, Virginia 22161(US)

(72) Inventor: **GALLO, Robert, C.**
**8513 Thornden Terrace**
**Bethesda, MD 20817(US)**
Inventor: **SALAHUDDIN, Syed, Zaki**
**12600 Newgate Road**
**Potomac, MD 20854(US)**
Inventor: **SAXINGER, Carl, W.**
**6814 Renita Lane**
**Bethesda, MD 20817(US)**
Inventor: **ABLASHI, Dharam, V.**
**4117 Barnsley Lane**
**Olney, MD 20832(US)**

(74) Representative: **Lamb, John Baxter et al**
**MARKS & CLERK**
**57-60 Lincoln's Inn Fields**
**London WC2A 3LS (GB)**

**Description**

A new DNA virus, designated Human B Lymphotropic Virus (HBLV), has been isolated from the blood leukocytes of patients with lymphoproliferative disorders. While the virus belongs morphologically to the Herpes family of viruses, HBLV, as shown below, this virus has not been previously characterized. HBLV is associated with some malignancies in AIDS and non-AIDS patients, but is distinctly different than Human T-cell Lymphotropic Virus Type III (HTLV-III), the causative agent of AIDS. HBLV contains a large double-stranded DNA genome, and selectively infects B cells; HTLV-III, on the other hand, contains a single stranded RNA genome, and selectively infects and is cytolytic for T-cells.

The nucleocapsid of the HBLV virus is of icosahedral symmetry with 162 capsomeres, and is enveloped in a lipid membrane. The outer surface of the viral envelope is covered with short spikes. The diameter of the enveloped virion is approximately 180 nm; the nucleocapsid is approximately 100 nm in diameter. The space between the capsid and the envelope, 35-40 nm, is filled with amorphous material. The nucleoprotein core or nucleoid is approximately 65 nm in diameter, and is occasionally rod-shaped or asymmetric.

Infection of primary cells or of cord blood cells produces characteristic large cells 4-10 days after infection. These cells are 2-4 times the diameter of small leukocytes, and exhibit cytopathogenic and cytolytic changes after about one week in culture. The nuclei of these cells is often highly convoluted, containing mainly euchromatic chromatin and nucleoli without remarkable features. Large numbers of virions are released by most infected cells.

The present invention relates to a method of detecting antibodies specific to Human B Lymphotropic Virus (HBLV) antigens in human sera. The preferred assays used in the detection of HBLV are Western Blot or immunofluorescent assay (IFA).

Human B Lymphotropic Virus (HBLV) is a newly discovered DNA virus isolated from the peripheral blood leukocytes of patients with various lymphoproliferative disorders. The ultrastructural characteristics of the HBLV virus, as well as its morphogenesis, place the virus in the family of Herpesviruses -- with similarities to and differences from any known members of the family. Immune electron microscopic studies show that patients from whom the virus has been isolated make highly specific antibodies to both the viral envelope and to internal components of the virus. Immunological, molecular, biological, and host range studies indicate that the HBLV virus has not been previously described.

Cultures of mononuclear cells from infected blood samples develop significant numbers of characteristic large cells 4-10 days after culture with primary cells or cord blood cells. Electron microscopy analysis shows that HBLV virus particles are present in large cells but absent in small lymphocytes. The infected cells are 2-4 times the diameter of small lymphocytes and do not show any initial obvious cytopathic changes. After one week in culture, however, cytopathic and cytolytic changes are readily observable. Specifically, the nuclei of infected cells are often highly convoluted; chromatin is mainly euchromatic and contain nucleoli without remarkable features. The cytoplasm displayed fairly large Golgi apparatus, vesicles of different sizes, prominent arrays of rough endoplasmic reticulum, and abundant mitochrondria. The general appearance of these cells is that of highly polymorphic proliferating blasts of lymphoid origin.

The main ultrastructural features of the HBLV virus are consistent with those of the Herpesvirus family. The nucleocapsid has an icosahedral symmetry with 162 capsomeres and is enveloped in a lipid membrane. The outer surface of the viral envelope is covered with short spikes. The diameter of the enveloped virion is approximately 180 nm; the diameter of the nucleocapsid is approximately 100 nm. The space between the capsid and the envelope, 35-40 nm, is primarily filled with amorphous material (this structure appears to be identical to the tegument described for several Herpesviruses). The nucleoprotein core or nucleoid is approximately 65 nm in diameter. Occasionally, nucleoids are rod-shaped or asymmetric.

Specific immunolabeling of extracellular virus occurs at the ultrastructural level using pre-absorbed patient's serum and an antiserum against human gamma globulin raised in goats, and labeled either with ferritin or with peroxidase. Large numbers of virions are released by most infected cells, and appear in tight clusters at the surface of the cells. Virtually all of the virions are labeled at their periphery. In some instances, the label penetrates into the virion, indicating that the envelopes of some of the virions are not intact and that some of the patient's serum contained antibodies to internal components of the virus as well as to the viral envelope.

The HBLV virus of the present invention is propagated by infecting human cord blood cells with HBLV, as is described in more detail in the Specific Disclosure.

2

DESCRIPTION OF THE FIGURES

Fig. 1 shows the immunofluorescent analysis of HBLV-infected enlarged cells (5 days post infection) exhibiting granular, nuclear, and cytoplasmic immunofluorescent staining. Small cells in the background (arrows) did not react with patient serum.

Fig. 2 shows peripheral blood leukocytes from a patient with AIDS-associated lymphoma (a) and HBLV-infected umbilical cord blood leukocytes (b) in cell culture.

SPECIFIC DESCRIPTION OF THE INVENTION

The present invention relates to a method of detecting antibodies specific to human B lymphotropic virus (HBLV) membrane antigens in human serum suspected of being infected with HBLV, comprising the steps of:

(1) providing HBLV infected human cord blood cells cells that have been

   (a) washed in serum-free medium,

   (b) separated from serum-free medium, and

   (c) resuspended in serum-free medium;

(2) incubating the resuspended HBLV infected cells with aliquots of each of

   (a) unknown serum sample,

   (b) known HBLV antibody-negative serum, and

   (c) known HBLV antibody-positive serum, in order to obtain a separate test, a separate negative control and a separate positive control first reaction mixtures;

(3) separating the cells from each of the first reaction mixtures from supernatant fluid and retaining the cells;

(4) washing the cells retained from the first reaction mixtures in serum-free medium;

(5) resuspending the cells obtained in step (4) in serum-free medium;

(6) separating the resuspended cells obtained in step (5) from supernatant fluid and retaining the cells;

(7) incubating the retained cells in step (6) with fluorescein isothiocyanate (FITC)-conjugated anti-human immunoglobulin antibody in order to obtain a separate test, a separate negative control and a separate positive control second reaction mixtures;

(8) separating the cells from each of the second reaction mixtures from supernatant fluid and retaining the cells;

(9) washing the cells retained from the second reaction mixtures in serum-free medium;

(10) resuspending the cells obtained in step (9) in serum-free medium;

(11) examining cells from step (10) for membrane fluorescence;

(12) comparing the fluorescence of test cells to the fluorescence of negative control and positive control cells; and

(13) determining the presence or absence of antibodies to HBLV membrane antigens by the presence or absence of fluorescent staining in the cells of step (11), respectively.

TABLE 1

| | No. of Patients | IF Assay | DOT Blot |
|---|---|---|---|
| AIDS* with B cell lymphoma (diffuse cell type) | 3 | + | + |
| Acute lymphocytic leukemia | 1 | + | + |
| Angio immuno-blastic lymphadenopathy | 2 | + | + |

*AIDS - acquired immune deficiency syndrome

The immunofluorescence assay which forms a part of the present invention is also used in detecting HBLV in fresh tissue cultures and in cultured peripheral cord blood cells and in the monitoring of infection in fresh human cord blood lymphocytes.

Sucrose gradient purification of HBLV. Heparinized peripheral blood leukocytes or human umbilical cord blood mononuclear cells are banded in Ficoll-Hypaque® and established in cell culture at 36°C following PHA-P (5μg/ml) stimulation for 48 hours. The cells are then grown in RPMI-1640 supplemented with 10% fetal bovine serum (heat inactivated, 56°C for 30 min.) and 5μg/ml hydrocortisone. Frozen supernatants obtained from the infected cells are thawed, collected in 250 ml tubes and spun at 3500 rpm in a Sorall GSA rotor at 5° C for 10 min. The clarified supernatants are transferred to SW28 tubes and spun and pelleted at 17,000 rpm for 90 min. at 5° C. Pellets obtained are resuspended in 10 mM Tris-HCl pH 7.4, 10 nM NaCl, 1 mM EDTA (TNE) to a volume of 300 microliters and layered onto a 15-60% sucrose gradient and spun in an SW41 rotor (Beckman) at 20,000 rpm for 30 min. at 5° C. Fractions of 1 ml are collected from the top of the gradient. Each fraction is diluted to 10 ml and spun and pelleted in an SW41 rotor at 17,000 rpm for 90 min. Pellets are resuspended in 300 microliters of TNE and aliquots assayed (by ELISA and Western Blot) for the presence of virus and for virus infectivity. Human B Lymphotropic Virus is easily detected in fractions 4-9 with a peak in fractions 5-7 by both assays. Extraction of nucleic acids from each fraction shows the presence of double stranded DNA in fractions 5-9 with a peak in fraction 7. Virus is also detected by electron microscopy in the SW41 gradient pellet as well.

Virus purified from fresh supernatants according to the above procedure is used for detailed electron microscopy.

Aliquots of the sucrose gradient fractions can be assayed for the presence of HBLV by DNA dot blot analysis using the pZVH14 insert as a probe. The pZVH14 molecular clone may be obtained from the American Type Culture Collection under Accession No. 40247.

The immunofluorescence assay and Western Blot assay are the preferred assays for detecting HBLV infection and HBLV antibodies in a variety of hematopoietic malignancies, including B-cell lymphomas of both AIDS and non-AIDS origin. The presence of HBLV antibodies is elevated in the following disease groups:

Burkitts lymphomas;

Hodgkin's disease;

A newly described infectious disease syndrome similar to that seen in Lake Tahoe characterized as an "acute mononucleosis-like syndrome" in adults; and

ALL as diagnosed in children of Caribbean and African origin.

HBLV Virus Propagation. Infection of human umbilical cord blood or peripheral blood mononuclear cells is conducted by cell-free transmission as follows:

1) Fresh blood samples are diluted 1:1 with RPMI-1640 and spun (and banded) on a Ficoll® gradient.

2) The banded molecular cells are washed and put into culture in the presence of PHA-P (5$\mu$g/ml) and HC (hydrocortisone) (5$\mu$g/ml) in 20% FCS (fetal calf serum) and RPMI-1640.

3) After 24 hours, Polybrene® (2$\mu$g/ml) is added to the culture and after 48 hours, the cells are pelleted.

4) A one ml aliquot of freshly harvested or frozen infected culture supernatant is added to the pellet and incubated at 37°C for 1 hour, with frequent agitation.

5) Fresh medium [10% FCS and HC (5$\mu$g/ml) in RPMI-1640] is then added to the suspension, cultured, and incubated at 36°C.

6) Within 2-10 days post infection, the characteristic enlarged refractile cells become visible. Supernatant is harvested at the peak of infection as measured by immunofluorescence and by visual observation of the culture for further transmission.

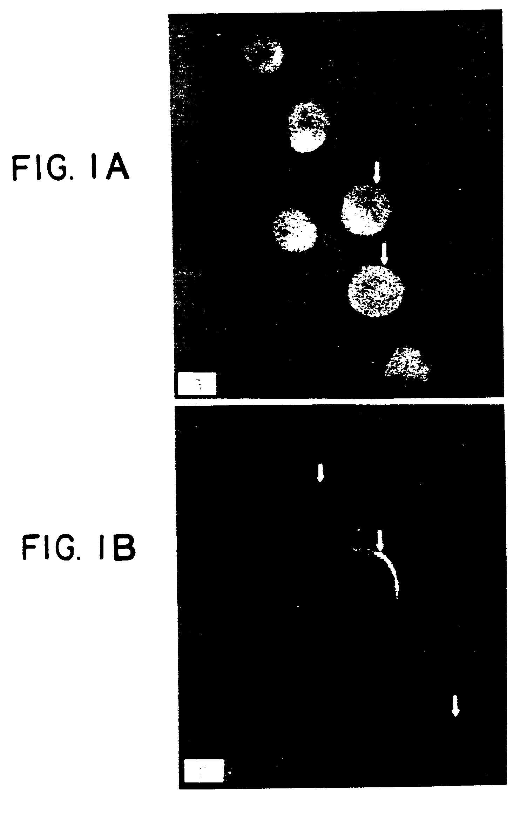

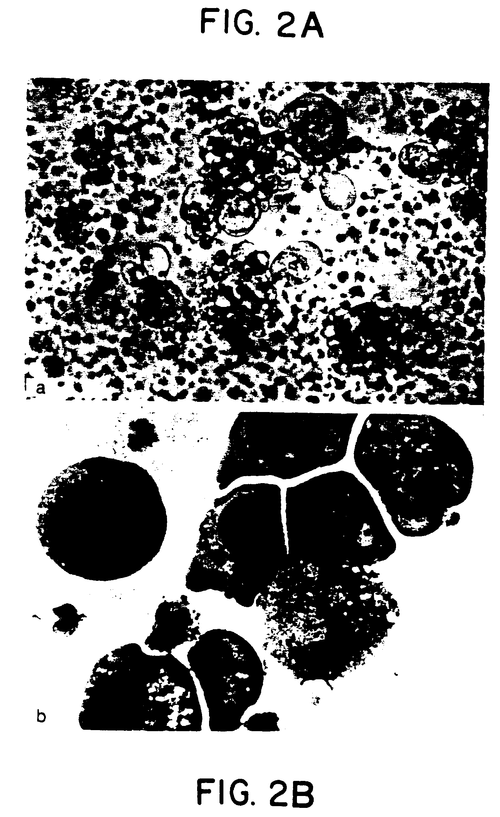

Detection of HBLV Infection in Cells. HBLV infected cells are maintained in the medium 1640 containing 10% FCS, 5$\mu$g/ml HC, and are incubated at 36°C. The uninfected cells are treated similarly, but are used as a control. The infection of HBLV in these cells is assayed using the IFA. A small cell sample is taken at different times and the cells are washed in PBS and stained with HBLV antibody positive serum and HBLV antibody negative serum. The infected cells are large cells (Fig. 2) showing first IF staining in the nucleus with HBLV antibody. These positive cells are mostly enlarged cells (See Figs. 1 and 2.) The control cells show no IF staining.

Detection of HBLV Antibodies. (a) Screening - Antibodies which specifically bind to HBLV are measured conveniently and sensitively using a dilution of infected cell cytoplasm deposited on a nitrocellulose membrane (Millitier, 96 well format) as the antigenic target. The binding of human antibody is quantified using alkaline phosphatase conjugated anti-human IgG and standard enzyme chemistries. The amount of cell cytoplasm and dilution of test antibody dilutions are determed by optimization of signal to noise ratios in preliminary experiments using negative control human antisera and uninfected cell cytoplasm as controls. The equivalent of 5,000 cells per well and test antibody dilutions of between 1/400 to 1/1600 dilutions are customarily used. Control wells consisting of uninfected cells of the same origin as the infected cells are included where highest specificity was required.

(b) Western Blot - Infected cell cytoplasm is separated by SDS-PAGE and transferred to a nitrocellulose membrane by standard methodology. Comparison of the binding of negative control sera and positive control sera to infected and non-infected cell proteins shows the presence of unique bands appearing only with infected cell cytoplasm and virus positive sera. Comparison of the nuclear, cytoplasmic, and cell culture supernatant fractions shows the highest concentration of antigens in the cell supernatant.

Indirect Immunofluorescence Assay. In conjunction with the procedures noted above for the isolation and propagation of HBLV, the following is an assay for HBLV which detects the IgG antibody to the late virus capsid antigens of Human B Lymphotropic Virus.

Human cord blood (CB) mononuclear cells are stimulated for 48 hours with PHA-P (5$\mu$g/ml) and after that, the cells are treated with Polybrene® (2$\mu$g/ml) for 48 hours. Primary human spleen cells are stimulated with PHA-P and then with Polybrene®. The cells are then infected with Human B Lymphotropic Virus as shown above.

HBLV fluid is harvested from the infected CB cells when they show >25-30% large giant cells; the cells are then tested by indirect immunofluorescence assay, using reference sera (positive and negative). Serum from one patient positive for HBLV infection, or other serum showing strong IFA reactivity, contains >1:80 titer to HBLV. The negative sera used in this procedure is at least two sera which have no antibody to HBLV. Uninfected cord blood cells are PHA-P stimulated for 48 hours. FITC affinity purified antihuman IgG (H and L) is prepared in goat or rabbit, at 1:15. Glass slides are teflon coated, with an uncoated 6 mm ID circle. IF mounting solution is used to affix the cells to the glass slides.

Procedure. Human cord blood cells infected with HBLV >25-30% englarged giant cells (>$10^5$/ml) are washed in PBS (without Ca + + and Mg + +) three times at 1000 rpm at 30 °C. The washed cells are put in a lymphocyte separating medium in order to remove any degenerating cells. The washed cells are suspended in PBS with Ca + + and Mg + + (>$10^5$ cells/ml) and deposited on the teflon slides. Similarly, uninfected CB cells are deposited on the slides and treated the same way as the infected cells. The cells are quickly air dried and then fixed in cold acetone (kept at 20° C) for 10 minutes at room temperature. The fixed cells may be stored at -20°C in a slide box to avoid moisture. All patients' sera to be tested is heat incubated at 56°C for 1/2 hour and then clarified to avoid precipitated material. The sera is then diluted at 1:10 twice in PBS (without Ca + + and Mg + +) using a shaker or rocker at 10 minute intervals. The slides are then air dried and FITC anti- human IgG is deposited on the slides, incubated for 40 minutes, washed three times, air dried, and mounted with IF mounting solution using a coverslip. The mounted slides may be kept at 4°C for one week with this mounting solution without loss of fluorescence.

The slides are read on an immunofluorescence scope and scored for positive and negative antibody titer. The positive serum showed bright green nuclear staining of enlarged cells with slightly dotted staining within the cell. The number of positive cells varies with the degree of infection. The negative sera show no staining to both the infected and uninfected cells. Similarly, positive serum show no IF staining on uninfected cells. The cells incubated with only FITC show no staining on infected and uninfected cells (see Figure 1a). The end point titer of the antibody to HBLV is based on either no staining or weak staining in the last dilution. The actual titer is calculated on the dilution of antibody showing faintly stained cells and the subsequent dilution being completely negative.

To detect viral membrane antigen (Figure 1b), HBLV-infected and uninfected live cells (nonfixed) are washed three times in serum-free medium and treated with patient's serum for 30 minutes at 4°C. The cells are again washed, treated with affinity-purified FITC anti-human IgG for another 30 minutes, washed in medium, and examined for membrane fluorescence as noted above. An HBLV-infected cell showing patchy surface fluorescence is shown in Figure 2b.

The following example illustrates one aspect of the present invention, in that a diagnostic test kit containing the specified reagents used in Example 1 is within the scope of the present invention.

EXAMPLES

Example 1. Detection of HBLV membrane antigents (MA) antibody in sera containing various diseases.

In the preparation of cells, HBLV infected viable cord blood (CB) cells were washed in serum free medium twice. Similarly, PHA-P stimulated uninfected CB cells were also washed and used as controls.

Reagents: (a) HBLV-MA antibody obtained from a patient containing virus capsid antibody; (b) HLBV negative antibody from normal healthy donor; (c) FITS antihuman IgG (A.P.).

Procedure:

(1) The washed HBLV infected and uninfected CB cells were centrifuged at 1000 rpm for 5 minutes.

(2) The centrifuged cells were suspended in serum-free medium at a density of <5 x $10^5$/ml.

(3) The suspended cells were added to various dilutions of unknown serum as well as other HBLV positive and negative reference sera. The cells were incubated either at 37°C for 45 minutes or 1/2 hour at 4°C.

5

(4) After the incubation, the cells were centrifuged at 1000 rpm, washed three times in serum-free medium and resuspended at a density of ≦1 x $10^5$/ml and centrifuged again in small tubes. After the centrifugation, the supernatant was discarded and FITC conjugate was added to the cells (1:15 dilution of the conjugate).

(5) The cells were incubated at 37°C for 45 minutes or 4°C for 1/2 hour.

(6) The cells were washed with serum free medium three times and kept resuspended at density of 1 x $10^5$/ml in the same medium.

(7) A drop containing suspended cells was deposited on a glass slide, and a glass cover slip was put on the cells and examined for membrane fluorescence.

Staining pattern. The MA positive cells showed bright greenish staining either full ring, or 1/2 rings or capping around the cell cytoplasm containing HBLV antigen (Fig. 1b). The dead cells normally picked up yellowish staining of the entire cell. The negative control cells were free of any staining.

Example 2.

TABLE 2

| Comparison of HBLV and Herpes Simplex Virus Morphology | | |
|---|---|---|
| Feature | HBLV | HSV |
| Diameter of Nucleoid | 60-80 nm | 50-70 nm |
| Diameter of Capsid | 95-105 nm | 95-100 nm |
| Symmetry of Capsid | Icosahedral | Icosahedral |
| No. of Capsomeres in Capsid | 162 | 162 |
| Thickness of Tegument | 25-40 nm | Often indistinct 25-40 nm |
| Diameter of Enveloped Virion | 160-200 nm | 150-220 nm |

Example 2 (Continued).

## TABLE 3

Comparison of Morphogenic Features of HBLV with Other Human Herpesviruses

Nuclear Features of Virus Infection

| Features | Characteristic of |
|---|---|
| Nucleocapsid assembly in the nucleoplasm | All |
| Tegument addition in the nucleoplasm | HBLV Only |
| Tegument addition at the nuclear membrane | All |
| Envelopment at the nuclear membrane | All |

Cytoplasmic and Extracellular Features of Virus Infection

| Features | Characteristic of |
|---|---|
| Enveloped virions in cisternae | All |
| Enveloped virions in cytoplasmic inclusions | CMV Only |
| Tegument-coated unenveloped nucleocapsids in cytosol | HBLV Only |
| Unenveloped nucleocapsids without tegument in cytosol | HSV-II Only |
| Enveloped extracellular virus | All |
| Unenveloped extracellular virus | All Except HBLV |

Example 3.

Monoclonal antibodies and hyperimmune sera prepared against human and simian herpesviruses were tested for reactivity with HBLV infected cells by indirect immunoflorescence procedures as described.

7

Monoclonal antibodies to EBV and HCMV were used at 1:40 dilution; HSV-I and II, VZV and HVS at a 1:10 dilution normal ascites fluid was used at 1:15 and 1:10 dilutions. Hyperimmue sera to African green and Rhesus monkey CMV were heat inactivated (50°C 30 min.) and clarified at 10,000 rpm and were used at 1:10 dilutions. In addition to the sera shown, human sera containing antibodies to EBV, CMV, HSV-I and II, and VZV also did not react with HBLV infected cells. African green and Rhesus sera containing antibody to CMV were also negative when tested with HBLV. Monoclonal antibodies to EBV, and HCMV, and ascites fluid from normal mouse were gifts from Dr. Gary Pearson, School of Medicine, Georgetown University, Washington, D.C. Monoclonal antibodies to VZV and HVS were obtained from Dr. Nancy Chung, Baylor College of Medicine, Houston, Texas, and Dr. John Dahlberg, NCI, Bethesda, Maryland, respectively. HSV-I and II monoclonal antibodies were purchased from Dupoint, Boston, MA. Hyperimmune sera to purified African green and Rhesus CMV were previously prepared in rabbits by Dr. Ablashi.

Abbreviations used: HBLV, Human B Lymphotropic Virus; EBV, Epstein-Barr Virus; HCMV, Human Cytomegalovirus; HSV, Herpes Simplex Virus; VZV, Varicella-zoster Virus; HVS, Herpes Virus Saimiri; VCA, Viral Capsid Antigen; MA, Membrane Antigen.

HBLV infected cord blood mononuclear cells were stained with an HBLV negative serum resulting in a considerable number of large cells with no immunofluorescence. The results are tabulated in Table 4.

Example 4.

Serum from old world and new world primates were tested for antibody to HBLV by indirect immunofluorescense as described.

Some sera from the old world primates were gifts from Dr. P. Kanki, Harvard School of Public Health, Boston, MA. All sera were heat inactivated at 56°C for 30 min., clarified by centrifugation before use. HBLV-infected cord blood leukocytes, P3HR-1 (an established cell line expressing EBV-VCA), and Owl monkey kidney cells infected by HSC-strain II were used for comparisons. When infected cells showed cytopathic effects, the cells were fixed in acetone and used for the IFA.

Three owl monkeys and one cottontop marmoset were previously innoculated with HVS. Sera from these animals possessed antibody to HVS late antigen which cross-reacted with Herpesvirus ateles. The results are tabulated in Table 5.

## TABLE 4

Immunological Cross Reactivities of HBLV to Other Human and Nonhuman Primates Herpesviruses Antibody Viruses Used to Infect Target Cells

| Source | HBLV | EBV | HCMV | HSV-I and II | VZV | HVS | Af. Gr. CMV | Rhesus CMV |
|---|---|---|---|---|---|---|---|---|
| EBV Monoclonal Antibody (VCA,EA,MA) | − | + | − | − | − | − | − | − |
| HCMV Monoclonal Antibody (VCA and EA) | − | − | + | − | − | − | − | − |
| HSV I and II Monoclonal Antibody (early and late antigens) | − | − | − | + | − | − | − | − |
| VZV Monoclonal Antibody (late antigens) | − | − | − | − | + | − | − | − |
| HVS Monoclonal Antibody (late antigens) | − | − | − | − | − | + | − | − |
| Af. Green Monkey CMV (hyperimmune serum) | − | − | − | − | − | − | + | − |
| Rhesus Monkey CMV (hyperimmune serum) | − | − | − | − | − | − | − | + |

EP 0 319 542 B1

## TABLE 5

### Cross-Reactivity of Nonhuman Primate Sera

Virus Used to Infected Target Cells

| Serum Sources | HBLV No. Positive/ No. Tested (Percent Positive) | EBV No. Positive (VCA)/No. Tested (Percent Positive) | HSV No. Positive/ No. Tested (Percent Positive) |
|---|---|---|---|
| **Old World Primates** | | | |
| Chimpanzee | 0/5 (0) | 5/5 (100%) | 0/4 (0) |
| Gorilla | 0/3 (0) | 2/3 (66.6%) | 0/3 (0) |
| Orangutan | 0/2 (0) | 1/2 (50%) | 0/2 (0) |
| Baboons | 0/3 (0) | 3/3 (100%) | 0/3 (0) |
| Stumptail | 0/2 (0) | 1/2 (50%) | 0/2 (0) |
| Rhesus | 0/9 (0) | 6/9 (66.6%) | 0/7 (0) |
| African Green | 0/10 (0) | 6/10 (60%) | 0/10 (0) |
| **New World Primates** | | | |
| Squirrel Monkeys | 0/10 (0) | 0/10 (0%) | 8/10 (80) |
| Owl Monkeys | 0/6 (0) | 0/6 (0%) | 3/6 (50) |
| Marmosets (common) | 0/6 (0) | 0/6 (0) | 0/6 (0) |
| Marmosets (cottontop) | 0/3 (0) | 0/3 (0) | 1/3 (33.3) |

## Claims

1. A method of detecting antibodies specific to human B lymphotropic virus (HBLV) membrane antigens in human serum suspected of being infected with HBLV, comprising the steps of:
   (1) providing HBLV infected human cord blood cells cells that have been
      (a) washed in serum-free medium,

EP 0 319 542 B1

(b) separated from serum-free medium, and

(c) resuspended in serum-free medium;

(2) incubating the resuspended HBLV infected cells with aliquots of each of

(a) unknown serum sample,

(b) known HBLV antibody-negative serum, and

(c) known HBLV antibody-positive serum, in order to obtain a separate test, a separate negative control and a separate positive control first reaction mixtures;

(3) separating the cells from each of the first reaction mixtures from supernatant fluid and retaining the cells;

(4) washing the cells retained from the first reaction mixtures in serum-free medium;

(5) resuspending the cells obtained in step (4) in serum-free medium;

(6) separating the resuspended cells obtained in step (5) from supernatant fluid and retaining the cells;

(7) incubating the retained cells in step (6) with fluorescein isothiocyanate (FITC)-conjugated anti-human immunoglobulin antibody in order to obtain a separate test, a separate negative control and a separate positive control second reaction mixtures;

(8) separating the cells from each of the second reaction mixtures from supernatant fluid and retaining the cells;

(9) washing the cells retained from the second reaction mixtures in serum-free medium;

(10) resuspending the cells obtained in step (9) in serum-free medium;

(11) examining cells from step (10) for membrane fluorescence;

(12) comparing the fluorescence of test cells to the fluorescence of negative control and positive control cells; and

(13) determining the presence or absence of antibodies to HBLV membrane antigens by the presence or absence of fluorescent staining in the cells of step (11), respectively.


**Patentansprüche**


**1.** Verfahren zum Nachweis von für ein Membran-Antigen des menschlichen B-lymphotropen Virus (HBLV) spezifischen Antikörpern in menschlichem Serum, das unter Verdacht steht, mit HBLV infiziert zu sein, mit den Schritten:

(1) Liefern von HBLV-infizierten, menschlichem Knochenmark entnommenen Blutzellen, die

(a) in einem serumfreien Medium gespült,

(b) von dem serumfreien Medium getrennt und

(c) in einem serumfreien Medium resuspendiert wurden;

(2) Inkubieren der resuspendierten HBLV-infizierten Zellen mit repräsentativen Proben von

(a) sowohl einer unbekannten Serum-Probe, als auch

(b) einem bekannten Serum ohne HBLV-Antikörper, als auch

(c) einem bekannten Serum mit HBLV-Antikörpern zum Erhalt von ersten Reaktionsmischungen eines separaten Tests, einer separaten negativen Kontrolle und einer separaten positiven Kontrolle;

(3) Trennen der Zellen von allen ersten Reaktionsmischungen von überschüssiger Flüssigkeit und Zurückbehalten der Zellen;

(4) Spülen der von den ersten Reaktionsmischungen zurückbehaltenen Zellen in einem serumfreien Medium;

(5) Resuspendieren der in Schritt (4) erhaltenen Zellen in einem serumfreien Medium;

(6) Trennen der in Schritt (5) erhaltenen resuspendierten Zellen von überschüssiger Flüssigkeit und Zurückbehalten der Zellen;

(7) Inkubieren der in Schritt (6) zurückbehaltenen Zellen mit einem Fluoreszein-Isothiozyanat-(FITC)-konjugierten Anti-Humanimmunoglobulin-Antikörper zum Erhalt von zweiten Reaktionsmischungen eines separaten Tests, einer separaten negativen Kontrolle und einer separaten positiven Kontrolle;

(8) Trennen der Zellen von allen zweiten Reaktionsmischungen von überschüssiger Flüssigkeit und Zurückbehalten der Zellen;

(9) Spülen der von den zweiten Reaktionsmischungen zurückbehaltenen Zellen in einem serumfreien Medium;

(10) Resuspendieren der in Schritt (9) erhaltenen Zellen in einem serumfreien Medium;

(11) Untersuchen der Zellen aus Schritt (10) auf Membranfluoreszenz;

(12) Vergleichen der Fluoreszenz der Testzellen mit der Fluoreszenz der Zellen der negativen Kontrolle und der positiven Kontrolle; und

(13) Bestimmen des Vorliegens von Antikörpern des HBLV-Membran-Antigens durch das vorliegen einer Fluoreszenzfärbung bei den Zellen aus Schritt (11) oder des Nichtvorliegens von Antikörpern des HBLV-Membran-Antigens durch das Nichtvorliegen einer Fluoreszenzfärbung bei den Zellen aus Schritt (11).

**Revendications**

1. Procédé de détection d'anticorps spécifiques des antigènes de membrane du virus lymphotrope B humain (HBLV) dans du sérum humain soupçonné d'être infecté par HBLV, comprenant les étapes consistant à :

(1) fournir des globules sanguins cordonaux humains qui ont été

(a) lavés dans du milieu exempt de sérum,

(b) séparés du milieu exempt de sérum et

(c) remis en suspension dans du milieu exempt de sérum ;

(2) incuber les globules infectés par HBLV, remis en suspension, avec des quantités aliquotes de respectivement

(a) un échantillon de sérum inconnu,

(b) un sérum connu ne réagissant pas aux anticorps HBLV et

(c) un sérum connu réagissant aux anticorps HBLV, pour obtenir un premier mélange réactionnel d'essai distinct, un premier mélange réactionnel témoin négatif distinct et un premier mélange réactionnel témoin positif distinct ;

(3) séparer les globules de chacun des premiers mélanges réactionnels du liquide surnageant et conserver les globules ;

(4) laver les globules conservés des premiers mélanges réactionnels dans du milieu exempt de sérum ;

(5) remettre en suspension les globules obtenus à l'étape (4) dans du milieu exempt de sérum ;

(6) séparer les globules remis en suspension, obtenus à l'étape (5), du liquide surnageant et conserver les globules ;

(7) incuber les globules conservés à l'étape (6) avec des anticorps anti-immuno-globuline humaine conjugués à de l'isothiocyanate de fluorescéine (FITC) pour obtenir un second mélange réactionnel d'essai distinct, un second mélange réactionnel témoin négatif distinct et un second mélange réactionnel témoin positif distinct ;

(8) séparer les globules de chacun des seconds mélanges réactionnels du liquide surnageant et conserver les globules ;

(9) laver les globules conservés des seconds mélanges réactionnels dans du milieu exempt de sérum ;

(10) remettre en suspension les globules obtenus à l'étape (9) dans du milieu exempt de Sérum ;

(11) examiner la fluorescence de membrane des globules de l'étape (10) ;

(12) comparer la fluorescence des globules testés à la fluorescence des globules témoins négatifs et des globules témoins positifs et

(13) déterminer la présence ou l'absence d'anticorps dirigés contre les antigènes de membrane de HBLV grâce à la présence ou à l'absence respectives de coloration fluorescente dans les globules de l'étape (11).

FIG. IA

FIG. IB

# FIG. 2A

# FIG. 2B